(19) Europäisches Patentamt
European Patent Office
Office européen des brevets

(11) Veröffentlichungsnummer: **0 032 553**
**B1**

# (12) EUROPÄISCHE PATENTSCHRIFT

(45) Veröffentlichungstag der Patentschrift: **06.04.83**

(51) Int. Cl.³: **C 07 F 15/06**, C 07 D 213/12

(21) Anmeldenummer: **80107665.4**

(22) Anmeldetag: **13.09.79**

(60) Veröffentlichungsnummer der früheren Anmeldung nach Art. 76 EPÜ:

(54) **Pi-Indenyl-(cycloocta-1,5-dien)-cobalt, pi-Trimethylsilyl-cyclopentadienyl-(cycloocta-1,5-dien)-cobalt, pi-Cyclopentadienyl-alpha,alpha'-bipyridyl-cobalt und Verfahren zu deren Herstellung sowie ihre Verwendung.**

(30) Priorität: **16.09.78 DE 2840460**

(43) Veröffentlichungstag der Anmeldung: **29.07.81 Patentblatt 81/30**

(45) Bekanntmachung des Hinweises auf die Patenterteilung: **06.04.83 Patentblatt 83/14**

(84) Benannte Vertragsstaaten: **AT BE CH DE FR GB IT LU NL SE**

(56) Entgegenhaltungen:
**DE-A-2 615 309**
**US-A-4 006 149**

**JOURNAL OF CHEMICAL SOCIETY, Dalton Transactions: Inorganic Chemistry (1979) FAIRHURST, G. et al. "Cyclopentadienyl-or pentamethylcyclopentadienyl-(arene)cobalt(III) complexes: arene = indole, benzene, mesitylene, hexamethylbenzene, 1,4-dihydroxy- and 1-hydroxy-4-methoxytetramethylbenzene",Seiten1531-8.**

**JOURNAL OF THE CHEMICAL SOCIETY, Dalton Transactions: Inorganic Chemistry (1979) GREEN, M.L.H. et al. "Some chemistry of ethyltetramethylcyclopentadienylcobalt: arene, ethylene, butadiene, amine, tertiary phosphine, and chloroderivatives", Seiten 355-60.**

(73) Patentinhaber: **Studiengesellschaft Kohle mbH, Kaiser-Wilhelm-Platz 1, D-4330 Mülheim/Ruhr (DE)**

(72) Erfinder: **Bönnemann, Helmut, Dr., Grashofstrasse 82, Essen (DE)**
Erfinder: **Samson, Marc, Dr., Bismarckstrasse 28, Mülheim/Ruhr (DE)**

(74) Vertreter: **von Kreisler, Alek, Dipl.-Chem. et al, Deichmannhaus am Hauptbahnhof, D-5000 Köln 1 (DE)**

(56) Entgegenhaltungen:
**JOURNAL OF THE CHEMICAL SOCIETY, Section A, (1971) London, GB: A. GRECO et al. "Synthesis and some reactions of pi-cyclo-octatrienyl-pi-cyclo-octatetra-enecobalt", Seiten 285-8.**

**SYNTHESIS, International journal of methods in synthetic organic chemistry, 1976, Nr. 1, Januar. WAKATSUKI, Y. et al. "Cobaltocene catalyzed synthesis of pyridines", Seiten 26-8, Communications.**

**TETRAHEDRON LETTERS, September 1973, Nr. 36 New York, USA: WAKATSUKI, Y. et al. "Cobaltcatalyzed synthesis of pyridines from acetylenes and nitriles", Seiten 3383-84.**

**SYNTHESIS, International journal of methods in synthetic organic chemistry, 1974, Nr. 8, August. BONNEMANN, H. et al. «Eine einfache, kobalt-katalysierte Pyridin-Synthese», Seiten 575-7.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

π-Indenyl-(cycloocta-1,5-dien)-cobalt, π-Trimethylsilyl-cyclopentadienyl-(cyclooacta-1,5-dien)-cobalt, π-Cyclopentadienyl-α,α'-bipyridyl-cobalt und Verfahren zu deren Herstellung sowie ihre Verwendung

Die vorliegende Erfindung betrifft π-Indenyl-(cycloocta-1,5-dien)-cobalt, π-Trimethylsilylcyclopentadienyl-(cycloocta-1,5-dien)-cobalt, π-Cyclo-pentadienyl-α,α'-bipyridyl-cobalt und Verfahren zu deren Herstellung sowie ihre Verwendung zur selektiven Herstellung von 2-Vinylpyridin aus Acetylen und Acrylnitril.

Die bis heute vorgeschlagenen Verfahren sind hinsichtlich der Katalysatorausnutzung und Produktausbeuten wirtschaftlich unbefriedigend, wie eine Prüfung der in Tabelle 1 zusammengefassten Daten zeigt.

Tabelle 1

| Literatur | Katalysator | Temp. °C | Nitril-Konzentration Mol/l | Verweilzeit Min. | Pyridin-Ausbeute, bez. auf Acrylnitrilumsatz | Produktbildungsschritte pro Co-Atom |
|---|---|---|---|---|---|---|
| DOS 2615309 C.P. Hardt Lonza AG Beispiel 3 | Cobaltocen | 120 | 4,57 | 60 | 41,6% | 38 |
| H. Yamazaki J. Wakatsuki Synthesis 1976, 26 pag. 28 | Cobaltocen | 110 | 5,05 | 420 | 31,0% | 48,0 |
| US-PS 4,006,149 Studienges. Kohle m.b.H. Beispiel 40 | Cyclopentadienyl Co-Cyclooctadien | 100 | 2,144 | 240 | 75,0% | 63,0 |

Yamasaki et al erhalten eine Ausbeute von 31% an 2-Vinylpyridin mit Cobaltocen als Katalysator. Die Synthese wird bei 110°C bei einer Reaktionsdauer von 7 Stunden und einer Konzentration an Acrylnitril von 5,05 Mol/l durchgeführt. Die Reaktion war mit Absicht bei einer deutlich niedrigeren Temperatur, nämlich 110°C, vergleichsweise zur Herstellung anderer Pyridinderivate ausgeführt worden, um den Verlust an Vinylpyridin durch Polymerisation herabzudrücken. Eine Verringerung der Reaktionsdauer auf 60 Minuten führt nach C.P. Hardt unter Verwendung des gleichen Katalysators, leicht erhöhter Temperatur (120°C) und leicht ermässigter Nitrilkonzentration (4,57 Mol/l) nicht zum Erfolg. Die Ausbeute bleibt unter 15%, d. h. für eine wirtschaftliche Verwertung unbefriedigend niedrig.

In der US-PS 4 006 149 sind Cobalt-I-Komplexverbindungen als Katalysatoren unter anderem für die Herstellung von Vinylpyridinen genannt, die vergleichsweise zu dem Katalysator, Cobaltocen, der vorgenannten Offenbarungen wesentlich wirkungsvoller sind. Bei 100°C und einer Verweilzeit von 4 Stunden werden bei einer Nitrilkonzentration von 2,14 Mol/l immerhin 75% Ausbeute erzielt. Bei der Wahl der vorgenannten Parameter – Absenkung der Temperatur und der Nitrilkonzentration, mittlerer Reaktionsdauer – war nicht zu erwarten, dass unter Verwendung von z. B. Cobaltocen als Katalysator eine wesentliche Verbesserung des Syntheseergebnisses erzielt werden konnte.

Die vorliegende Erfindung betrifft die folgenden Gegenstände:

π-Indenyl-(cycloocta-1,5-dien)-cobalt,
π-Trimethylsilyl-cyclopentadienyl-(cycloocta-1,5-dien)-cobalt,
π-Cyclo-pentadienyl-α,α'-bipyridyl-cobalt,

ein Verfahren zur Herstellung von π-Indenyl-(cycloocta-1,5-dien)-cobalt, das dadurch gekennzeichnet ist, dass man den Natta-Komplex $C_8H_{13}CoC_4H_6$ mit Inden umsetzt und anschliessend das Reaktionsprodukt mit Cyclooctadien-1,5 umsetzt und die erhaltene Verbindung abtrennt.

Das Verfahren zur Herstellung von π-Trimethylsilylcyclopentadienyl-(cycloocta-1,5-dien)-cobalt ist dadurch gekennzeichnet, dass man Cyclopentadien mit Butyllithium versetzt, anschliessend Trimethylsilylchlorid zugibt, das erhaltene Trimethylsilylcyclopentadien mit $Co_2(CO)_8$ umsetzt und das erhaltene $Me_3SiCpCo(CO)_2$ in Cyclooctadien-1,5 erhitzt und das erhaltene Produkt isoliert.

Das Verfahren zur Herstellung von π-Cyclopentadienyl-α,α'-bipyridyl-cobalt ist dadurch gekennzeichnet, dass man Cyclopentadienylcobaltdicarbonyl mit α,α'-Bipyridyl umsetzt und das erhaltene Reaktionsprodukt isoliert. Die vorstehend genannten Verbindungen werden zur katalytischen selektiven Herstellung von 2-Vinylpyridin aus Acetylen und Acrylnitril verwendet.

Beim Einsatz der erfindungsgemässen Verbindungen als Katalysator für die selektive Herstellung von 2-Vinylpyridin aus Acetylen und Acrylnitril können folgende Verfahrensbedingungen angewandt werden.

Für die Wahl der Temperatur ist der Bereich um 150–160°C vorteilhaft, ein Temperaturbereich von 140 – 180°C aber auch anwendbar.

Die Reaktionsdauer liegt bei etwa 5 Minuten bis zu höchstens 50 Minuten, kann aber auch im Bereich unterhalb 5 Minuten bis zu einer Grössenordnung von Sekunden liegen, wenn entsprechende Reaktionsapparaturen, wie z. B. Strömungsrohre, verwendet werden. Vorteilhaft ist im allgemeinen eine Reaktionsdauer von 15 bis 30 Minuten.

Die Konzentration an Acrylnitril liegt in einem Bereich von etwa 0,1 Mol/l bis zu 2 Mol/l oder auch darüber. Vorzugsweise liegt die Konzentration an Acrylnitril um oder über 1 Mol/l.

Als Lösungsmittel sind solche geeignet, die gegenüber den Reaktionspartnern im angegebenen Temperaturbereich inert sind. Solche sind beispielsweise aliphatische Lösungsmittel und insbesondere aromatische Lösungsmittel wie Benzol oder Toluol.

Zweckmässig führt man die Umsetzung in einem Druckgefäss mit guter Durchmischungseinrichtung durch, damit während der Umsetzung eine Sättigung der Lösung mit Acetylen gewährleistet wird. Der Acetylendruck liegt dabei zwischen 5 und 20 bar, vorzugsweise zwischen 8 und 17 bar.

Das Verfahren kann diskontinuierlich aber alternativ auch kontinuierlich durchgeführt werden, so entweder in einer Rührkesselkaskade oder im Strömungsrohr, wobei eine rasche Abkühlung des Auslaufstromes sichergestellt sein sollte. Der Katalysator wird zweckmässigerweise in Form vorgefertigter Cobaltverbindungen eingesetzt, er kann aber auch in der Reaktionsmischung in situ hergestellt werden.

Beispiel 1

Darstellung von π-Indenyl-(cycloocta-1,5-dien)-Cobalt

$C_8H_{13}CoC_4H_6$ + $C_9H_8$ → $C_9H_7Co$ (dien)

(222) (116)

Natta-Komplex Inden

$C_9H_7Co$ (dien) + COD-1,5 → $C_9H_7CoCOD$

(108) (284)

Ind Co COD

Man versetzt 3,8 g (17,1 mMol) Natta-Komplex, gelöst in 100 ml Pentan, mit 1,98 g (17,1 mMol) Inden bei −30°C. Danach wird die Reaktionslösung langsam auf RT gebracht, und man lässt noch 20 h nachreagieren. Anschliessend wird ca. 5 ml COD-1,5 zugegeben. Die klare rote Lösung wird ca. 24 h zum Rückfluss erhitzt. Nach Abkühlen wird evtl. entstandener Niederschlag abfiltriert und die Lösung auf ca. 30 ml eingeengt und langsam bis auf −50°C gekühlt. Es entstehen braunrote Kristalle. Die überstehende Mutterlauge drückt man ab und trocknet die Kristalle (rotbraungefärbte Nadeln) 1 h bei RT im Hochvakuum.

Ausbeute: 2,4 g (50% d. Th.)

Smp.: 98°C

Elementaranalyse:

| | | | |
|---|---|---|---|
| Gef.: | C 73,20% | H 6,86% | Co 19,29% |
| Ber.: | C 72,34% | H 6,78% | Co 20,88% |

IR-Analyse:

1450; 1325; 1315; 1200; 1030; 960; 905; 840; 790; 735; 725 $cm^{-1}$

$^1$H-NMR ($d_8$-Toluol, 80 MHz):

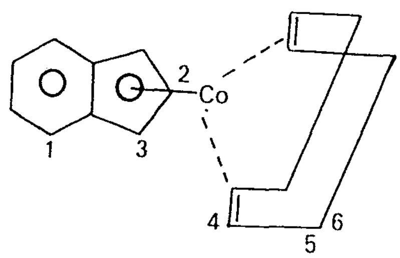

$\delta H_1$: 7,14 (s)
$\delta H_2$: 5,78 (t, J = 2,4 Hz)
$\delta H_3$: 3,89 (d, J = 2,4 Hz)
$\delta H_4$: 3,40 (m)
$\delta H_5$: 2,09 (m)
$\delta H_6$: 1,40 (m)

$^{13}$C-NMR ($d_8$-Toluol):

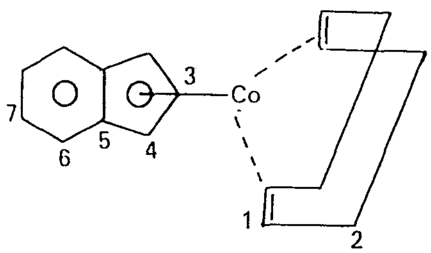

$\delta C_1$: 67,38 (d, $J_{C,H}$ = 152 Hz)
$\delta C_2$: 31,35 (t, $J_{C,H}$ = 127 Hz)
$\delta C_3$: 88,51 (d, $J_{C,H}$ = 174,6 Hz)
$\delta C_4$: 75,80 (d, $J_{C,H}$ = 174,4 Hz)
$\delta C_5$: 105,48 (s)
$\delta C_6$: 124,27 (d, $J_{C,H}$ = 159 Hz)
$\delta C_7$: 122,98 (d, $J_{C,H}$ = 161 Hz)

Massenspektrum:

m/e: 282 ($M^+$, 100%); 252 (42%); 174 (80%); 164 (25%); 138 (28%); 124 (30%); 115 (45%); 59 (15%);

Lit.:
«π-Indenyl-(cyclooctatrien)-cobalt», A. Greco, M. Green, F. G. Stone, J. Chem. Soc. (A), 286 (1971)»

Beispiel 2
Darstellung von π-Trimethylsilylcyclopentadienyl-(cycloocta-1,5-dien)-Cobalt

CpH + n-BuLi → LiCp + n-Butan
(66)

(1) LiCp + $Me_3SiCl$ → $Me_3SiCp$ + LiCl
(72) (108,5) (138)

(2) 2 $Me_3SiCp$ + $Co_2(CO)_8$ → 2 $Me_3SiCpCo(CO)_2$ + 4 CO + $H_2$
(138) (342) (252)

(3) $Me_3SiCpCo(CO)_2$ + COD-1,5 → $Me_3SiCpCoCOD$ + 2 CO
(252) (108) (304)

(1) 33 g (0,5 Mol) Cyclopentadien werden bei Raumtemperatur vorgelegt und mit 200 ml Benzol und 100 ml Tetrahydrofuran verdünnt. Innerhalb von 2 h werden 213 ml einer 15proz. Lösung Butyllithium in Hexan zugetropft. Abschliessend werden 64,5 g (0,5 Mol) Trimethylsilylchlorid, gelöst in 100 ml Benzol, zugetropft und die Reaktionsmischung 15 h unter Rückfluss gekocht. Nach Abkühlen wird der entstandene weisse Niederschlag (LiCl) abfiltriert und mit Benzol nachgewaschen. Das Filtrat wird eingeengt und anschliessend destilliert.
Sdp.: 60–65°C/Wasserstrahlpumpe ca. 18 mbar
Ausbeute: 30 g (46,5% d.Th.)

(2) Zu einer Lösung von 3,42 g (10 mMol) $Co_2(CO)_8$ in 20 ml Äther werden ca. 10 ml Trimethylsilylcyclopentadien gegeben. Die Mischung wird 1 h bis zum Rückfluss erhitzt. Nach Filtration wird das Lösungsmittel abgezogen und der Rückstand destilliert.
Sdp.: 50–52°C/0,7 mbar
Ausbeute: 4,3 g (85% d.Th.)

(3) Eine Lösung von 3,90 g (14,8 mMol) $Me_3SiCpCo(CO)_2$ in 10 ml COD-1,5 wird 12 h bis zum Rückfluss erhitzt. Nach Abkühlen wird die Lösung eingeengt, und man erhält als Rückstand eine hellbraune kristalline Masse. Umkristallisation aus Pentan bei −40°C liefert das reine Produkt
Ausbeute: 3,6 g (80% d.Th.)
Smp.: 64°C

Elementaranalyse:

| | | | | |
|---|---|---|---|---|
| Gef.: | C 64,54% | H 7,98% | Co 18,53% | Si 8,82% |
| Ber.: | C 63,16% | H 8,55% | Co 19,41% | Si 9,04% |

IR-Analyse:
1440; 1360; 1315; 1240; 1155; 1055; 1040; 900; 875; 850; 820(s); 800; 740; 680; 620 $cm^{-1}$.

$^1H$-NMR ($d_8$-Toluol, 60 MHz)

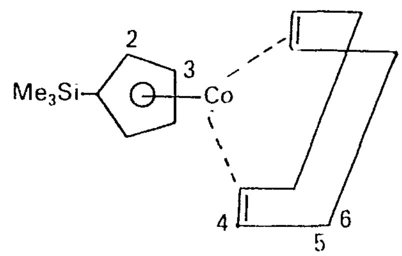

$\alpha H_1$: 0,0 (s), innerer Standard
$\alpha H_2$: 3,0 (t, J = 2 Hz)
$\alpha H_3$: 4,38 (t, J = 2 Hz)
$\alpha H_4$: 1,22 (m)
$\alpha H_5$: 2,0 (m)
$\alpha H_6$: 3,0 (m)

Massenspektrum:
m/e: 304 ($M^+$, 45%); 229 (100%); 196 (40%); 181 (18%); 137 (15%); 124 (15%); 107 (20%); 93 (10%); 73 (18%)

Lit.:

1. Darstellung $Me_3SiCpH$: K. C. Frisch, J. Am. Chem. Soc. 75, 6050 (1953).
2. Darstellung $Me_3SiCpCo(CO)_2$: E. W. Abel, S. Moorehouse, J. Organomet. chem. 28, 211–215 (1971).
3. Allgemeine Lit. SiCp-Met.-Verbindungen: «Advances in Organometallic Chemistry», Vol. 15 (1977), I. Haidue, V. Popa, s. 113 (Academic Press).


Beispiel 3
Darstellung von π-Cyclopentadienyl-α,α′-bipyridyl-Cobalt

(1) $CpCo(CO)_2$ + α,α′-Bipyridyl → CpCobipy + 2 Co
(180) (156) (280)

(2) $CpCo(CH_2{=}CH_2)_2$ + α,α′-Bipyridyl → CpCobipy + 2 $CH_2{=}CH_2$
(180) (156) (280)

(1) Zu einer Lösung von 8,15 g (45,3 mMol) Cyclopentadienylcobaltdicarbonyl in 100 ml Xylol wird bei RT eine Lösung von 7,06 g (45,3 mMol) α,α'-Bipyridyl in 50 ml Xylol zugetropft. Anschliessend wird bis zum Rückfluss erhitzt. Der Fortschritt der Reaktion ist daran zu erkennen, dass abnehmende Mengen $CpCo(CO)_2$ mit dem Lösungsmittel mitgerissen werden bis schliesslich der Rückfluss farblos kondensiert; ca. 12 h Reaktionszeit bei 160°C. Die Farbe ändert sich während der Reaktion von tiefrot nach tiefviolett. Abkühlen und Einengen des Lösungsmittels liefert einen schwarzvioletten Niederschlag, der aus Pentan bei −50°C umkristallisiert wird.
Ausbeute: 10,7 g (84% d. Th.)

Elementaranalyse:
Gef.: C 64,01% H 5,6% N 10,03% Co 20,90%
Ber.: C 64,30% H 4,68% N 10,00% Co 21,03%

Massenspektrum:
m/e: 280 ($M^+$, 100%); 215 (89%); 189 (10%); 162 (10%); 156 (29%); 140 (14%); 130 (14%); 59 (20%)

Die folgenden Beispiele zeigen die Anwendung der erfindungsgemässen Verbindungen als Katalysatoren für die selektive Herstellung von 2-Vinylpyridin aus Acetylen und Acrylnitril.

Beispiel 4

Eine Lösung von π-Indenyl-cyclooctadien-1,5-cobalt in Toluol wird mit Acrylnitril in den angegebenen Mengen versetzt. Die Lösung wird bei 20°C in einen 500 ml Edelstahl-Autoklaven mit magnetischer Rühreinrichtung eingesaugt. Man sättigt die Lösung bei 7 bar mit Acetylen und heizt innerhalb 15 Minuten auf 150°C auf, wobei der Druck durch Zupressen von Acetylen auf 17 bis 18 bar erhöht wird. Verbrauchtes Acetylen ergänzt man laufend. Nach 30 Minuten Verweilzeit bei 150°C bringt man das Druckgefäss binnen 10 Minuten durch äussere Kühlung auf 30°C. Das Rohprodukt wird aus dem Autoklaven ausgefüllt und die flüchtigen Bestandteile abkondensiert, wobei ein Rückstand verbleibt. Die eingesetzten Mengen, die Bedingungen sowie das Ergebnis sind aus dem Folgenden ersichtlich:

Eingesetzt:
0,1492 g (0,529 mMol) Indenyl-Cobalt-COD
26,20 g (494,4 mMol) Acrylnitril (1,65 molar)
231,25 g Toluol

Bedingungen:
Reaktionstemperatur: 140°C
Verweilzeit bei 140°C: 20 Minuten
Reaktionsaustrag: 271,15 g
Kondensat: (0,25 mbar): 268,3 g
Rückstand: 2,6 g
Ergebnis (lt. gaschromatographischer Analyse):
16,79 g nicht umgesetztes Acrylnitril
16,44 g 2-Vinylpyridin

Ausbeute, bez. auf umgesetztes Acrylnitril: 88% 2-Vinylpyridin
Katalysatorausnutzung: 294 Mol Pyridin/g At Cobalt

Beispiel 5

101,4 mg (0,3595 mMol) π-Indenyl-Cobalt-COD löst man in 151,8 g Toluol auf und versetzt mit 19,0 g (358,5 mMol) Acrylnitril, wobei 200 ml einer 1,79 molaren Nitrillösung einschliesslich Katalysator resultieren. Diese Lösung wird bei 20°C in einem 500-ml-Edelstahlautoklaven eingesaugt. Man sättigt die Lösung mit 7 bar Acetylen. Innerhalb von 12 Minuten heizt man auf 145°C auf, wobei der Druck durch Zupressen von Acetylen auf 18,5 bar erhöht und konstant gehalten wird.

Nach 17 Minuten Reaktionszeit dosiert man 105,5 mg (0,374 mMol) Indenyl-Cobalt-COD, 29,9 g Toluol gelöst, innerhalb von 6 Minuten zu. Nach 18 Minuten Reaktionszeit bei 145°C kühlt man das Druckgefäss binnen 10 Minuten auf 25°C ab.

Reaktionszeit: 41 Minuten
Reaktionstemperatur: 145°C
Reaktionsaustrag: 210,7 g
Kondensat (0,25 mbar): 206,6 g
Rückstand: 2,9 g

Ergebnis (lt. gaschromatographischer Analyse).
9,31 g nicht umgesetztes Acrylnitril
15,4 g 2-Vinylpyridin

Ausbeute, bez. auf umgesetztes Acrylnitril: 79% 2-Vinylpyridin
Katalysatorausnutzung: 197 Mol Pyridin/g At Cobalt

Beispiel 6

Darstellung von 2-Vinylpyridin mit π-Trimethylsilylcyclopentadienyl-(cycloocta-1,5-dien)-cobalt als Katalysator

Eingesetzt:
0,222 g (0,730 mMol) $Me_3SiCpCoCOD$
15,55 g (293,40 mMol) Acrylnitril (1,13 molar)
208,65 g Toluol

Bedingungen:
Reaktionstemperatur: 150°C
Reaktionszeit: 30 Min.
Reaktionsaustrag: 233,55 g
Kondensat (0,25 mbar): 231 g
Rückstand: 2,0 g

Ergebnis (lt. gaschromatographischer Analyse):
9,51 g Acrylnitril (nicht umgesetzt)
10,94 g 2-Vinylpyridin

Ausbeute, bez. auf umgesetztes Acrylnitril: 91% 2-Vinylpyridin
Katalysatorenausnutzung: 143 Mol Pyridin/g At Cobalt

Beispiel 7

Darstellung von 2-Vinylpyridin mit π-Cyclopenta-

dienyl-(α,α'-Bipyridyl)-cobalt als Katalysator

Eingesetzt:
0,150 g (0,536 mMol) CpCobipy
16,70 g (315,1 mMol) Acrylnitril (1,21 molar)
209,25 g Toluol

Bedingungen:
Reaktionstempertur: 145°C
Reaktionszeit: 15 Min.
Reaktionsaustrag: 233,50 g
Kondensat (0,25 mbar): 229,80 g
Rückstand: 1,5 g

Ergebnis (lt. gaschromatographischer Analyse):
10,55 g Acrylnitril (nicht umgesetzt)
9,82 g 2-Vinylpyridin

Ausbeute, bez. auf umgesetztes Acrylnitril: 81% 2-Vinylpyridin
Katalysatorausnutzung: 174 Mol Pyridin/g At Cobalt

**Patentansprüche**

1. π-Indenyl-(cycloocta-1,5-dien)-cobalt.
2. π-Trimethylsilylcyclopentadienyl-(cycloocta-1,5-dien)-cobalt.
3. π-Cyclopentadienyl-α,α'-bipyridyl-cobalt.
4. Verfahren zur Herstellung von π-Indenyl-(cycloocta-1,5-dien)-cobalt, dadurch gekennzeichnet, dass man den Natta-Komplex $C_8H_{13}CoC_4H_6$ mit Inden umsetzt und anschliessend das Reaktionsprodukt mit Cyclooctadien-1,5 umsetzt und die erhaltene Verbindung abtrennt.
5. Verfahren zur Herstellung von π-Trimethylsilylcyclopentadienyl-(cycloocta-1,5-dien)-cobalt, dadurch gekennzeichnet, dass man Cyclopentadien mit Butyllithium versetzt, anschliessend Trimethylsilylchlorid zugibt, das erhaltene Trimethylsilylcyclopentadien mit $Co_2(CO)_8$ umsetzt und das erhaltene $Me_3SiCpCo(CO)_2$ in Cyclooctadien-1,5 erhitzt und das erhaltene Produkt isoliert.
6. Verfahren zur Herstellung von π-Cyclopentadienyl-α,α'-bipyridyl-cobalt, dadurch gekennzeichnet, dass man Cyclopentadienylcobaltdicarbonyl mit α,α'-Bipyridyl umsetzt und das erhaltene Reaktionsprodukt isoliert.
7. Verwendung der Verbindungen nach Ansprüchen 1–3 zur katalytischen, selektiven Herstellung von 2-Vinylpyridin aus Acetylen und Acrylnitril.

**Claims**

1. π-Indenyl (cycloocta-1,5-diene)cobalt.
2. π-Trimethylsilylcyclopentadienyl (cycloocta-1,5-diene)cobalt.
3. π-Cyclopentadienyl-α,α'-bipyridylcobalt.
4. A process for the preparation of π-indenyl (cycloocta-1,5-diene)cobalt, characterized in that the Natta complex $C_8H_{13}CoC_4H_6$ is reacted with indene, and the reaction product is subsequently reacted with cycloocta-1,5-diene and the obtained compound is separated.
5. A process for the preparation of π-trimethylsilylcyclopentadienyl(cycloocta-1,5-diene)cobalt, characterized in that butyllithium is added to cyclopentadiene, trimethylsilyl chloride is subsequently added thereto, the obtained trimethylsilyl cyclopentadiene is reacted with $Co_2(CO)_8$, and the obtained $Me_3SiCpCo(CO)_2$ is heated in cycloocta-1,5-diene and the obtained product is isolated.
6. A process for the preparation of π-cyclopentadienyl-α,α'-bipyridylcobalt, characterized in that cyclopentadienyl cobalt dicarbonyl is reacted with α,α'-bipyridyl and the obtained reaction product is isolated.
7. Use of the compounds according to any one claims 1 to 3 for the catalytic, selective preparation of 2-vinylpyridine from acetylene and acrylonitrile.

**Revendications**

1. Pi-indényl-(cyclooctadiène-1,5)-cobalt.
2. Pi-triméthylsilylcyclopentadiényl-(cyclooctadiène-1,5)-cobalt.
3. Pi-cyclopentadiényl-α,α'-bipyridyl-cobalt.
4. Procédé pour la préparation de Pi-indényl-(cyclooctadiène-1,5)-cobalt, caractérisé en ce que l'on fait réagir le complexe de Natta $C_8H_{13}CoC_4H_6$ sur l'indène, puis que l'on fait réagir le produit de la réaction sur du cyclooctadiène-1,5 et que l'on sépare le composé obtenu.
5. Procédé pour la préparation de Pi-triméthylsilylcyclopentadiényl-(cyclooctadiène-1,5)-cobalt, caractérisé en ce que l'on additionne le cyclopentadiène de butyllithium, en ce que l'on ajoute ensuite du chlorure de triméthylsilyle, que l'on fait réagir le triméthylsilylcyclopentadiène obtenu sur du $Co_2(CO)_8$, que l'on chauffe le $Me_3SiCpCo(CO)_2$ obtenu dans du cyclooctadiène-1,5 et que l'on isole le produit obtenu.
6. Procédé pour la préparation de Pi-cyclopentadiényl-α,α'-bipyridyl-cobalt, caractérisé en ce que l'on fait réagir du cyclopentadiénylcobaltdicarbonyle sur de l'α,α'-bipyridyle et que l'on isole le produit de réaction obtenu.
7. Utilisation des composés selon les revendications 1 à 3 pour la préparation sélective catalytique de 2-vinylpyridine à partir d'acétylene et d'acrylonitrile.